# EUROPEAN PATENT APPLICATION

(11) **EP 4 521 597 A1**
(43) Date of publication of application: **12.03.2025**
(21) Application number: 24194147.5
(22) Date of filing: 12.08.2024
(51) Int. Cl.: H02J 9/06, A61B 6/03, A61B 6/00

(54) **METHODS AND SYSTEMS FOR POWER SUPPLY**

(30) Priority: 30.08.2023 US 202318458736
(71) Applicant: GE Precision Healthcare LLC, Waukesha, WI 53188 (US)
(72) Inventor: GUO, Hexin, Beijing, 100176 (CN); YANG, Xuyong, Beijing, 100176 (CN); MIRZAEI, Saeid, Waukesha, 53188 (US); DHUNGANA, Prajjwal, Waukesha, 53188 (US)
(74) Representative: Kilburn & Strode LLP

(57) **Abstract**

Various methods and systems are provided for a power supply system. In one example, a method and system includes a power distribution unit (PDU) configured to receive power from a main power source and an uninterruptible power supply (UPS), wherein the UPS is configured to directly power an output alternating current (AC) load after the main power source in unavailable, and the UPS is further configured to power an output high voltage direct current (HVDC) load via passing current through an autotransformer to boost the voltage and an AC/DC converter.

## Description

### TECHNICAL FIELD

Embodiments of the subject matter disclosed herein relate to providing power to medical imaging system in response to a main power source being absent due to a utility power outage.

### BACKGROUND

A computerized tomography (CT) imaging system may receive AC power from a main power source, such as a utility power source. The utility power source may be connected to a utility grid. During some conditions, the main power source may not be provided (i.e., may not be availble for use by the CT imaging system) in response to power outages, component failures or due to the main power source being unexpectedly cut-off.

Certain components within a CT imaging system, such as an X-ray source or X-ray Tube may need to cool down during a shutdown before the main power source is turned off from the CT imaging system. Unexpected power outages may damage certain components, such as an X-ray source or X-ray tube in the CT imaging system. To protect these components and extend their life, it may be desired to provide back-up power during unexpected power outages of the main power source in order to maintain a cool down routine of the components even when the main power source is not available.

### SUMMARY

This summary introduces concepts that are described in more detail in the detailed description. It should not be used to identify essential features of the claimed subject matter, nor limit the scope of the claimed subject matter.

In one aspect, a method and system includes a power distribution unit (PDU) configured to receive power from a main power source and an uninterruptible power supply (UPS), wherein the UPS is configured to directly power an output alternating current (AC) load after the main power source is unavailable, and the UPS is further configured to power an output high voltage direct current (HVDC) load via passing current through an autotransformer to boost the voltage and an AC/DC converter.

In another aspect, a computed tomography (CT) imaging system, includes a gantry coupled to an output HVDC load a power cabinet coupled to an output AC load, and a power distribution unit (PDU). The PDU is configured to receive power from one of a main power source and an uninterruptible power supply (UPS), wherein the UPS provides power to the HVDC load via an autotransformer when the main power source is unavailable. A plurality of contactors and a timer are coupled to the PDU and UPS. A controller with computer readable instructions stored on memory thereof controls the plurality of contactors based on availability of the main power source and an input from the timer.

In yet another aspect, a method for a computer tomography (CT) imaging system, executed via instructions stored on memory of a controller, includes in response to a main power source being unavailable, powering an output AC load directly via an uninterruptible power supply (UPS), waiting for a time delay, and powering an output HVDC load via power coupled from the UPS via an autotransformer to boost a voltage output from the UPS. In response to the main power source becoming available, the system executes instructions including waiting for a tube of the CT imaging system to cool, and powering the output HVDC load via the main power source.

The brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings.
FIG. 1 shows a pictorial view of a computed tomography (CT) imaging system, according to an embodiment.
FIG. 2 shows a block diagram of an exemplary CT imaging system, according to an embodiment.
FIGS. 3A-4B show schematic diagrams of a series of operations of the power circuitry under normal operating conditions.
FIGS. 5A-8B show schematic diagrams of a series of operations of the power circuitry and control circuitry under power loss operating conditions.
FIGS. 9-10B show schematic diagrams of a series of operations of the power circuitry and control circuity power return conditions.
FIG. 11 shows a method for switching a power supply to the CT imaging system in response to an availability of power from the main power source and controlling return of the CT imaging system to the main power source.

### DETAILED DESCRIPTION

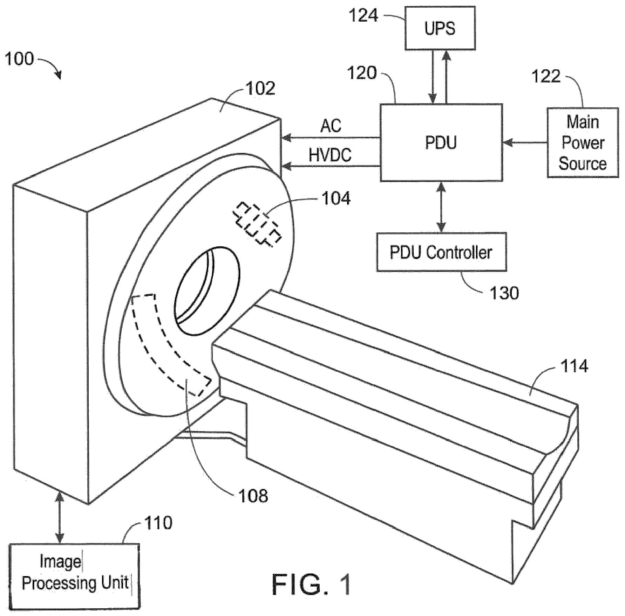

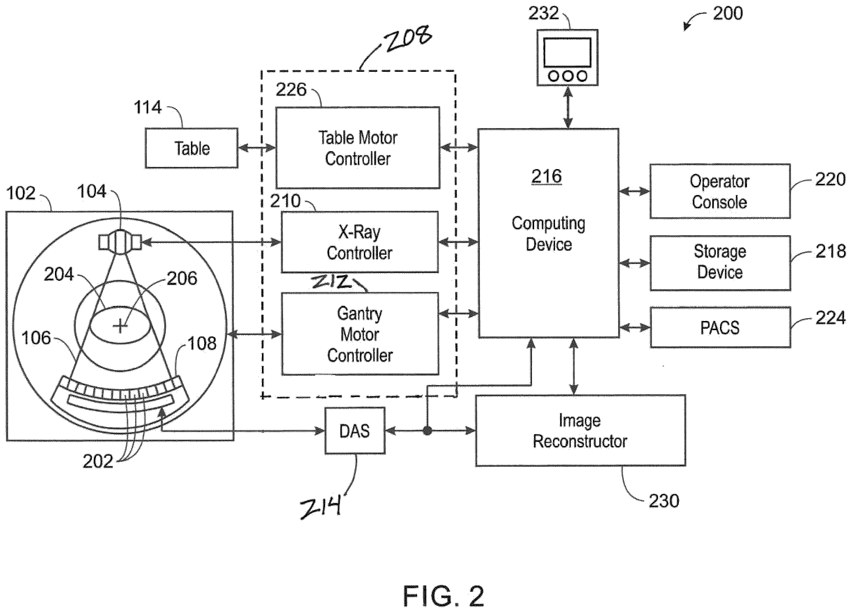

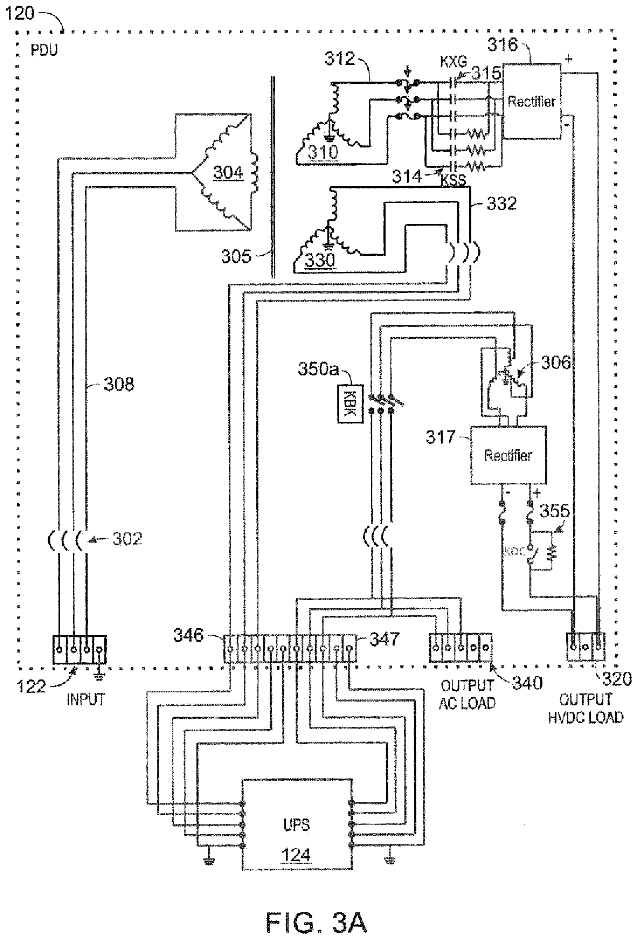

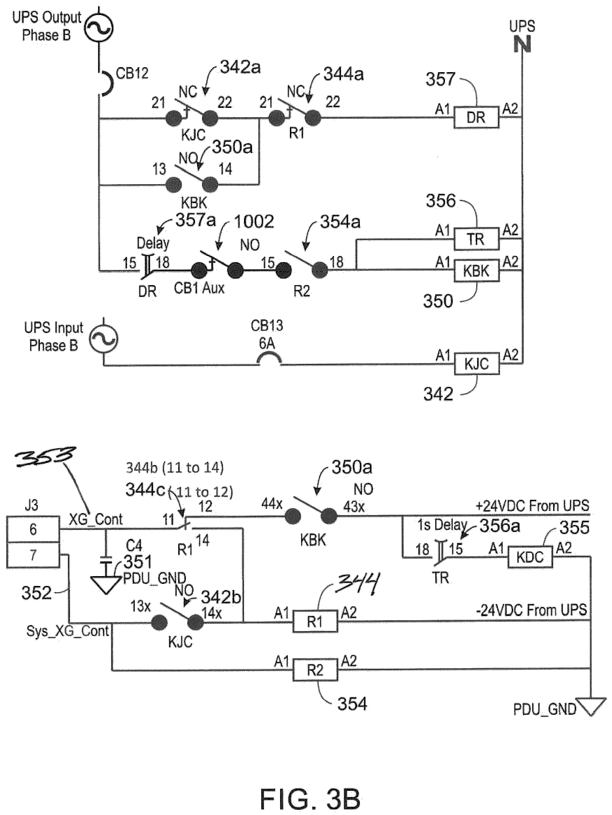

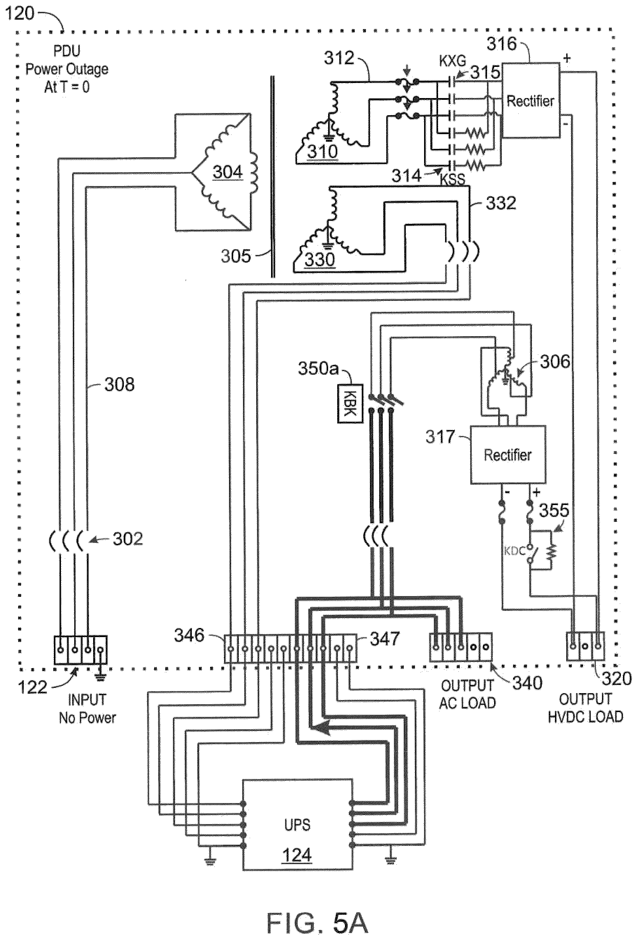

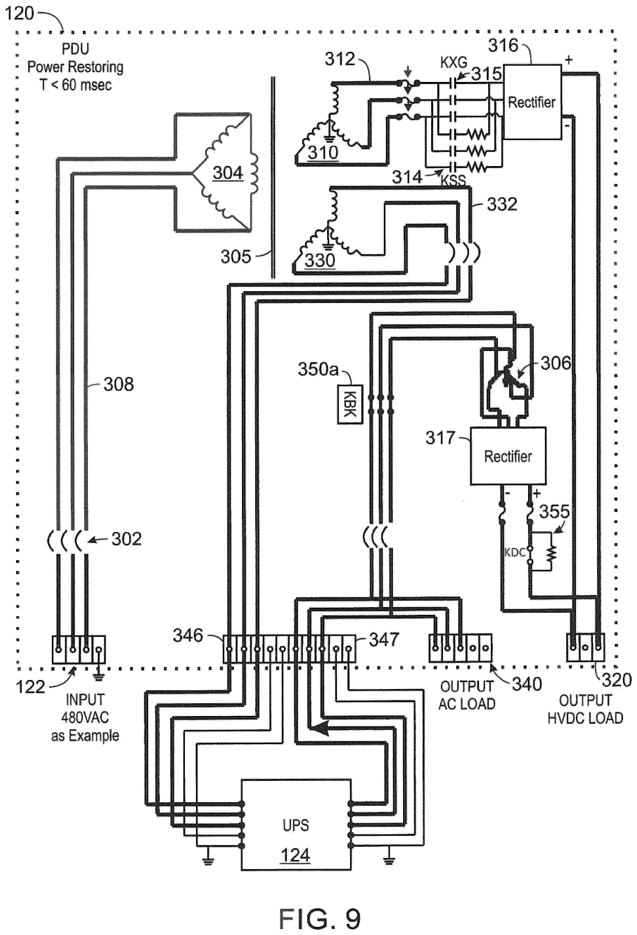

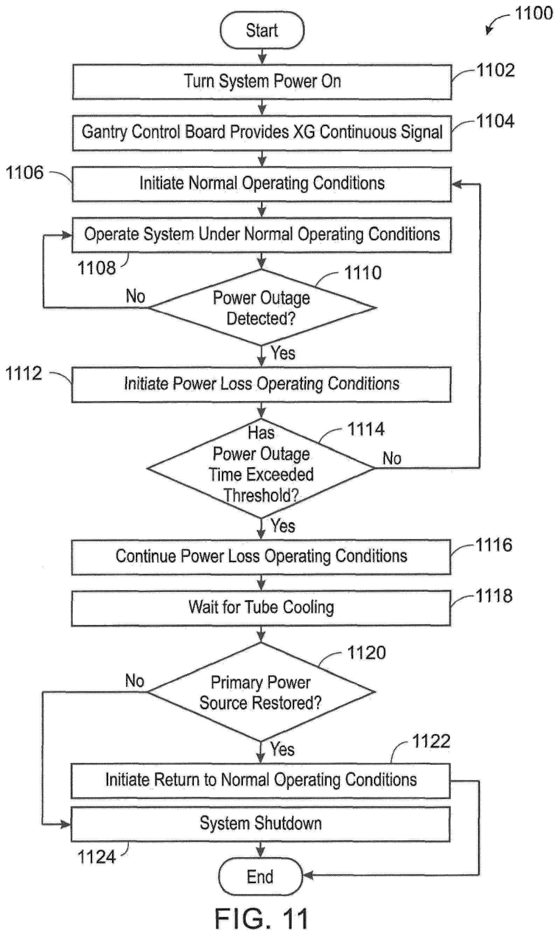

The following description relates to embodiments of a back-up power system for an imaging system, as illustrated in FIGS. 1 and 2. The back-up power system for the imaging system may be configured as an uninterruptible power supply (UPS) coupled to a power distribution unit (PDU) of the imaging system. FIGS. 3-4B depict the illustration of an electrical circuitry coupling between a main power source or source providing alternating current (AC) power and an uninterruptible power supply (UPS) to a power distribution unit (PDU) in normal operating conditions from turning on the CT imaging system until the system is ready to perform an imaging scan. FIGS. 5A-8B depict the illustration of the electrical circuitry and control circuitry during a power loss condition during which the power source does not provide power to the imaging system and the UPS provides backup power. FIGS. 9-10B depict the illustration of the electrical circuity and control circuitry during a power return condition, during which the system changes from using the UPS for power to using the power source after the power source has been restored. A method for operating the UPS based on an availability of power from a main power source is shown in FIG. 11.

In one example of the present disclosure, the imaging system may include a gantry. The gantry may include an X-ray tube bearing, such as a liquid bearing, which may be cooled following operation. To execute a cooling routine of the X-ray tube bearing in the gantry, power, such as electrical energy, may be consumed. In an event where power is not supplied to the imaging system (i.e., is not availble for use by the CT imaging system due to, for example, power loss or disconnect from a power source), shutdown of the gantry power may occur without cooling the bearing, which may result in degradation and/or a reduced useful life.

In many applications, the imaging system may be arranged proximate to a UPS system configured to provide back-up power to a computer and a console of the imaging system. However, the UPS system is not wired to provide power to the gantry to enable a desired cooling prior to shut down in the event where a main power source is interrupted (e.g., absent). Furthermore, the gantry may not be sized to power the computer, the console, and the gantry for an extended period of time.

The inventors have recognized these drawbacks and come up with ways to adjust one or more electrical circuits between the UPS and the PDU to supply power from the UPS to the gantry when the main power source is unavailable. By doing this, an extra power source apart from the pre-existing UPS is not needed, which may decrease manufacturing and installation costs while also decreasing a packaging size of the system. For example, by adding multiple contactors (e.g., switches), a source of the power supply may be reliably controlled such that both supplies (e.g., the UPS and the main power source) are not providing power simultaneously.

FIGS. 1 to 10B show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

Though a CT imaging system is described by way of example, it should be understood that the present methods and systems may also be useful when applied to other imaging systems, such as X-ray imaging systems, magnetic resonance imaging (MRI) systems, positron emission tomography (PET) imaging systems, single-photon emission computed tomography (SPECT) imaging systems, ultrasound imaging systems, and combinations thereof (e.g., multi-modality imaging systems, such as PET/CT, PET/MR or SPECT/CT imaging systems). The present discussion of a CT imaging system is provided merely as an example of one suitable imaging system.

FIG. 1 illustrates an exemplary CT imaging system 100 configured for CT imaging. Particularly, the CT imaging system 100 is configured to image a subject such as a patient, an inanimate object, one or more manufactured parts, and/or foreign objects such as dental implants, stents, and/or contrast agents present within the body. In one embodiment, the CT imaging system 100 includes a gantry 108, which in turn, may further include at least one X-ray source 104 configured to project a beam of X-ray radiation 106 (see FIG. 2). Specifically, the X-ray source 104 is configured to project the X-ray radiation beams 106 towards an X-ray detector array 108 positioned on the opposite side of the gantry 108. Although FIG. 1 depicts only a single X-ray source 104, in certain embodiments, multiple X-ray sources and detectors may be employed to project a plurality of X-ray radiation beams 106 for acquiring projection data at different energy levels corresponding to the patient. In some embodiments, the X-ray source 104 may enable dual-energy gemstone spectral imaging (GSI) by rapid peak kilovoltage (kVp) switching. In some embodiments, the X-ray detector employed is a photon-counting detector which is capable of differentiating X-ray photons of different energies. In other embodiments, two sets of X-ray sources and detectors are used to generate dual-energy projections, with one set at low-kVp and the other at high-kVp. It should thus be appreciated that the methods described herein may be implemented with single energy acquisition techniques as well as dual energy acquisition techniques.

In certain embodiments, the CT imaging system 100 further includes an image processor 110 configured to reconstruct images of a target volume of the subject using an iterative or analytic image reconstruction method. For example, the image processor 110 may use an analytic image reconstruction approach such as filtered back projection (FBP) to reconstruct images of a target volume of the patient. As another example, the image processor 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject. As described further herein, in some examples the image processor 110 may use both an analytic image reconstruction approach such as FBP in addition to an iterative image reconstruction approach.

In some CT imaging system configurations, an X-ray source projects a cone-shaped X-ray radiation beam which is collimated to lie within an X-Y-Z plane of a Cartesian coordinate system and generally referred to as an "imaging plane." The X-ray radiation beam passes through an object being imaged, such as the patient or subject. The X-ray radiation beam, after being attenuated by the object, impinges upon an array of detector elements. The intensity of the attenuated X-ray radiation beam received at the detector array is dependent upon the attenuation of an X-ray radiation beam by the object. Each detector element of the array produces a separate electrical signal that is a measurement of the X-ray beam attenuation at the detector location. The attenuation measurements from all the detector elements are acquired separately to produce a transmission profile.

In some CT imaging systems, the X-ray source and the detector array are rotated with a gantry within the imaging plane and around the object to be imaged such that an angle at which the X-ray radiation beam intersects the object constantly changes. A group of X-ray radiation attenuation measurements, e.g., projection data, from the detector array at one gantry angle is referred to as a "view." A "scan" of the object includes a set of views made at different gantry angles, or view angles, during one revolution of the X-ray source and detector.

The projection data is processed to reconstruct an image that corresponds to a two-dimensional slice taken through the object or, in some examples where the projection data includes multiple views or scans, a three-dimensional rendering of the object. One method for reconstructing an image from a set of projection data is referred to in the art as the filtered back projection technique. Transmission and emission tomography reconstruction techniques also include statistical iterative methods such as maximum likelihood expectation maximization (MLEM) and ordered-subsets expectation-reconstruction techniques as well as iterative reconstruction techniques. This process converts the attenuation measurements from a scan into integers called "CT numbers" or "Hounsfield units," which are used to control the brightness of a corresponding pixel on a display device.

To reduce the total scan time, a "helical" scan may be performed. To perform a "helical" scan, the patient is moved while the data for the prescribed number of slices is acquired. Such a system generates a single helix from a cone beam helical scan. The helix mapped out by the cone beam yields projection data from which images in each prescribed slice may be reconstructed.

As used herein, the phrase "reconstructing an image" is not intended to exclude embodiments of the present invention in which data representing an image is generated but a viewable image is not. Therefore, as used herein, the term "image" broadly refers to both viewable images and data representing a viewable image. However, many embodiments generate (or are configured to generate) at least one viewable image.

The CT imaging system 100 may receive power from a main power source 122 or from an uninterruptible power source (UPS) 124 through a power distribution unit (PDU) 120. Additionally or alternatively, the CT imaging system may receive power from a generator, wherein power from the generator may be provided through a similar coupling as the main power source 122. In one example, the PDU 120 may include one or more sensors configured to sense an availability of power from the main power source 122. A PDU controller 130 may be configured to receive feedback from the plurality of sensors and adjust a position of one or more actuators in response to the availability of power from the main power source 122 as well as command signals from a gantry control board (GCB) in the CT imaging system 102. The one or more actuators may be adjusted after a specified time delay is measured by a timer. In one example, the one or more actuators are contactors and/or switches, configured to alternate between the main power source 122 and the UPS 124 based on the availability of power from the main power source 122. In one example, if power from the main power source is unavailable, then the PDU controller 130 may signal to actuate a first switch to break a circuit in which the main power source 122 is arranged and to actuate a second switch to complete a circuit in which the UPS 124 is arranged such that power is supplied from the UPS 124 to the CT imaging system, as will be described in greater detail below.

The PDU controller 130 may include instructions stored on memory thereof that when executed cause the PDU controller 130 to adjust switches or contactors controlling power received from the main power source 122 when power from the main power source is unavailable. Power from the main power source 122 may be detected via a current sensor. Feedback from the current sensor may prompt the PDU controller 130 to actuate switches from the main power source 122 while actuating a UPS switch (after a time delay) from the UPS to output loads of the PDU 120 to power the CT imaging system 100.

FIG. 2 illustrates an exemplary imaging system 200 similar to the CT imaging system 100 of FIG. 1. In accordance with aspects of the present disclosure, the imaging system 200 is configured for imaging a subject 204. In one embodiment, the imaging system 200 includes the detector array 108 (see FIG. 1). The detector array 108 further includes a plurality of detector elements 202 that together sense the X-ray radiation beam 106 (see FIG. 2) that pass through the subject 204 (such as a patient) to acquire corresponding projection data. Accordingly, in one embodiment, the detector array 108 is fabricated in a multi-slice configuration including the plurality of rows of cells or detector elements 202. In such a configuration, one or more additional rows of the detector elements 202 are arranged in a parallel configuration for acquiring the projection data.

In certain embodiments, the imaging system 200 is configured to traverse different angular positions around the subject 204 for acquiring desired projection data. Accordingly, the gantry 108 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the X-ray source 104 and the detector array 108 rotate, the detector array 108 collects data of the attenuated X-ray beams. The data collected by the detector array 108 undergoes pre-processing and calibration to condition the data to represent the line integrals of the attenuation coefficients of the scanned subject 204. The processed data are commonly called projections.

In some examples, the individual detectors or detector elements 202 of the detector array 108 may include photon-counting detectors which register the interactions of individual photons into one or more energy bins. It should be appreciated that the methods described herein may also be implemented with energy-integrating detectors.

The acquired sets of projection data may be used for basis material decomposition (BMD). During BMD, the measured projections are converted to a set of material-density projections. The material-density projections may be reconstructed to form a pair or a set of material-density map or image of each respective basis material, such as bone, soft tissue, and/or contrast agent maps. The density maps or images may be, in turn, associated to form a volume rendering of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 108 and the operation of the X-ray source 104. In some embodiments, the control mechanism includes a timer 209. In certain embodiments, the control mechanism 208 further includes an X-ray controller 210 configured to provide power and timing signals to the X-ray source 104. Additionally, the control mechanism 208 includes a gantry motor controller 212 configured to control a rotational speed and/or position of the gantry 108 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into so-called macro-detectors, as described further herein. The data sampled and digitized by the DAS 214 is transmitted to a computer or computing device 216. In one example, the computing device 216 stores the data in a storage device or mass storage 218. The storage device 218, for example, may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the X-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates only one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 108 for acquiring projection data corresponding to the target volume of the subject 204.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

In one embodiment, the display 232 allows the operator to evaluate the imaged anatomy. The display 232 may also allow the operator to select a volume of interest (VOI) and/or request patient information, for example, via a graphical user interface (GUI) for a subsequent scan or processing.

In one example, the UPS 124 of FIG. 1 may be a preexisting UPS of the computing device 216 and auxiliary components thereof (e.g., the display 232, the operator console 220, etc.). As will be described herein, the inventors have found a way to modify one or more circuits of the PDU 120 and the UPS 124 of FIG. 1 such that the UPS 124 previously configured to only power the computing device 216 when the power from the main power source 122 was unavailable to also power the gantry 108. When main power is available, the UPS may be charged via the main power source. As such, a desired cooling of the gantry 108 may be executed in response to an event where power from the main power source 122 is unavailable.

Turning now to FIGS. 3A-4B an embodiment of a power interface of the UPS device 124 and the main power source 122 with the PDU 120 is depicted. As such, components previously introduced may be similarly numbered in these figures. The power distribution unit 120 may include a main power source 122, a circuit having a breaker 302, a three phase input 304 for the power, and a transformer 305. The transformer 305 may have a primary winding which is integrated with the three phase input, a first secondary winding 310 and a second secondary winding 330. The power distribution unit 120 may include a plurality of electrical lines 308 extending through corresponding switches in the three phase input 304 to the primary winding of transformer 305. The breaker 302 may be configured to trip in response to 150A (amps) or more of current flowing through any of the plurality of electric lines 308.

The primary winding of transformer 305 includes an electric winding configured to draw power from the plurality of electrical lines or wires. The winding may be electrically coupled to windings of a first secondary winding 310 and a second secondary winding 330.

The first secondary winding 310, which may include a higher voltage than the second secondary winding 330, may direct power, via a plurality of electric wires 312, to a rectifier 316. In some examples, the plurality of electrical lines or wires 312 may each include fuses configured to disrupt the circuit in response to a current flow through the plurality of electrical lines or wires exceeding a rating of the fuses. A plurality of contacts (e.g., KXG contact 315, KSS contact 314) may be positioned between the first secondary winding 310 and the rectifier 316. The rectifier 316 may be a passive or active rectifier, configured to convert alternating current (AC) to direct current (DC). The DC lines or wires are coupled to an output HVDC load 320, which may be used to supply power to a gantry (e.g., gantry 108 of FIG. 1). In one example, electrical power supplied to the output HVDC load 320 is a relatively high voltage (e.g., greater than 600V DC).

The second secondary winding 330 may direct power, via a plurality of electrical lines or wires 332, to an output AC load 340, via the UPS 124. Each of the plurality of electrical lines or wires 332 may include a fuse rated to disrupt the circuit in response to an electrical current exceeding a rating of the fuse. In one example, the fuses are rated to 50A. However, the fuses may be rated to other amperages based on a sizing and amp rating of the plurality of electric wires. The output AC load 340 may send power to lower power demand devices, such as a console, power cabinet, computer, and the like.

FIG. 3A shows a schematic of the PDU 120 which includes a three phase main transformer 305, auto-transformer 306, and two AC to DC converters 316 and 317 and some three phase contactors: KSS 314, KXG 315, KBK 350a and KDC 355. FIG. 3B depicts a control circuit associated with the schematic of FIG. 3A. The control circuit, which is shown in FIG. 3B, includes some three phase contactors: KJC 342, 342a, 342b; KBK 350, 350a; KDC 355, 350a; some timers: DR 357, 357a; TR 356, 356a; and some relays: R1 344, 344a, 344b (the common contact 11 is connected to contact 14), 344c (the common contact 11 is connnected to contact 12); and R2 354, 354a. The main transformer 305 has three windings, primary winding 304 is a Delta connection and connected to the main utility power source 122 by the main circuit breaker (CB1) 302. The first and second seconday windings 310, 330 are Wye connections. The first seconday winding 310 provides High Voltage Direct Current (HVDC) power to the therminal blocks (TS2) 320. The second secondary winding 330 provides Low Voltage Alternative Current (LVAC) power to the terminal blocks (TS5) 340. All of the componets depicted in FIG. 3A & 3B are positioned inside of the PDU 120 except the UPS 124. The UPS 124 may be optional, and can be used as a backup power for the PDU 120. The input power of the UPS 124 is connected to a terminal block 346 of the PDU 120 and the output power of the UPS 124 is connected to a terminal block 347 of the PDU 120. If the UPS is not avalible as backup power, the terminal block 346 must be connected to the terminal block 347 directly.

In FIG. 3B, the control circuitry recieves power from the output phase B of the UPS 124 and via a 24V supply line. Additionally, the input phase B of the UPS 124 provides 120V to the control circuitry at single phase (e.g., Phase B) is used to provide power to the control circuitry because only one phase is needed to enalbe the control circuitry to determine whether a power outage has occurred, and to trigger the response to the power outage. Specifically, the KJC contact 342 is the trigger for the respones to the power outage because the KJC contact is operative to detect the loss of power from the UPS 124 via Phase B. The power could come from either Phase A or Phase C instead. The control circuitry is also connected to the gantry control board (GCB) via terminals 6 and 7.

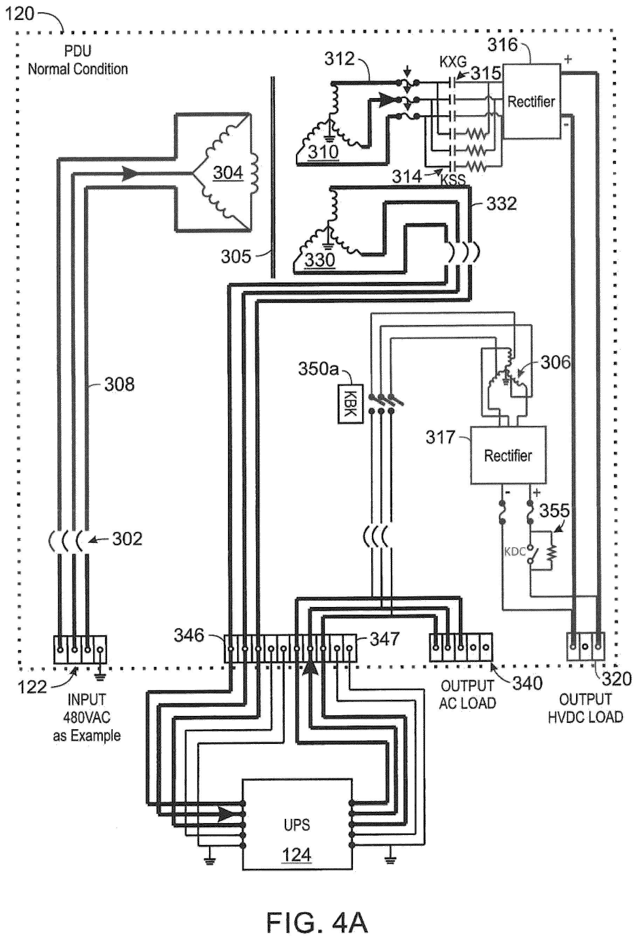

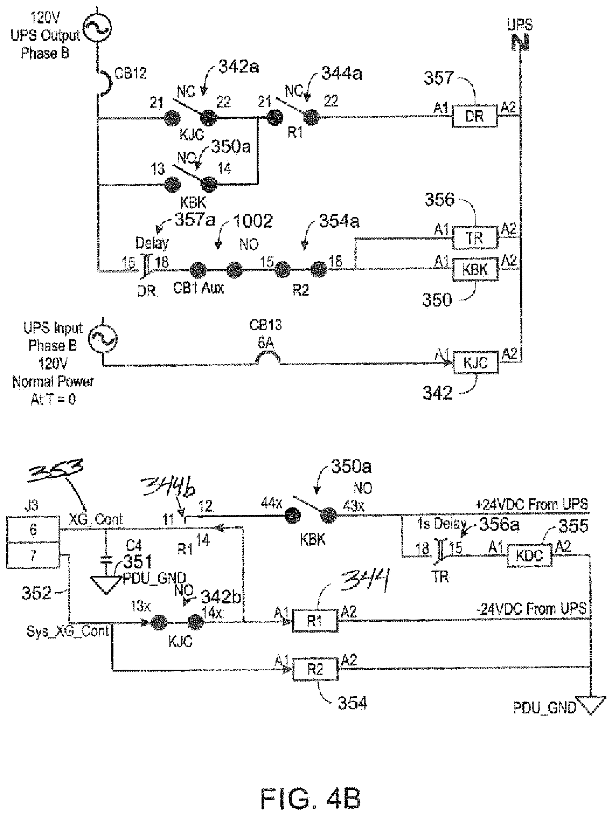

FIGS. 3A-4B show schematic diagrams of a series of operations of the power circuitry under normal operating condition. FIGS. 3A and 4A show both the power circuits and FIGS. 3B and 4B depict the control circuits. FIGS. 3A and 3B depict the first stage of operation during normal operating conditions (e.g., receiving power from the power source). During normal operating condition, the normaly closed (NC) KJC contact 342a and R1 contact 344a, 344c are closed at an initial startup phase. When the power is provided from the power source 122 to the PDU and transferred to the second secondary winding 330. It is presented to the terminal block 346 of the PDU 120, the NC of KJC contact 342a opens, while the normaly open (NO) of KJC auxiliary contact 342b closes, as shown in FIGS. 4A and 4B. The KJC contact 342 is a contact operative to detect whether the main power source is available. When the main power source 122 is available, the KJC contact or switch 342, 342a, 342b is in a first position (e.g., NC of KJC contact 342a is open, NO of KJC auxiliary contact 342b is closed), which enables normal operating conditions. When the main power source 122 is not available, such as prior to the system being powerd on or during a power outage condition, the KJC contact or switch 342 is in a second position (e.g., NC of KJC contact 342a is closed, NO of KJC auxiliary contact 342b is open). At the noraml operating condition, power flows through the UPS 124 by the terminal block 346 of the PDU 120 and from the UPS back to the terminal block 347 of the PDU 120, and to the AC power output or load 340, PDU TS5. Additionally, power flows from the UPS 124 to a KBK contact 350a, but the KBK contactor 350a is disconnected during normal operating condition. As shwon in FIGS. 4A and 4B, the gantry control board (GCB) sends a signal (e.g., a SYS_XG_CONT signal 352) to the PDU 120. The XG_CONT signal 353 provides singal power to contacts to keep contacts (e.g., a KSS contact 314, and after short delay, a KXG contact 315) in a closed position when the contacts would be normally open. Next, the R1 contact 344 is switched, an R2 contact 354a is closed, and the KSS 314 contact is closed, as shown in FIG. 4A. After a short delay (e.g., 1.2 seconds), the KXG contact 315 is closed, as shown in FIG. 4A. HVDC power is then supplied to the terminal blocks TS2 320 via the the recitifier 316, and normal operation of the CT imaging system resumes, as depicted in FIG. 4A. The system is now ready to perform scans.

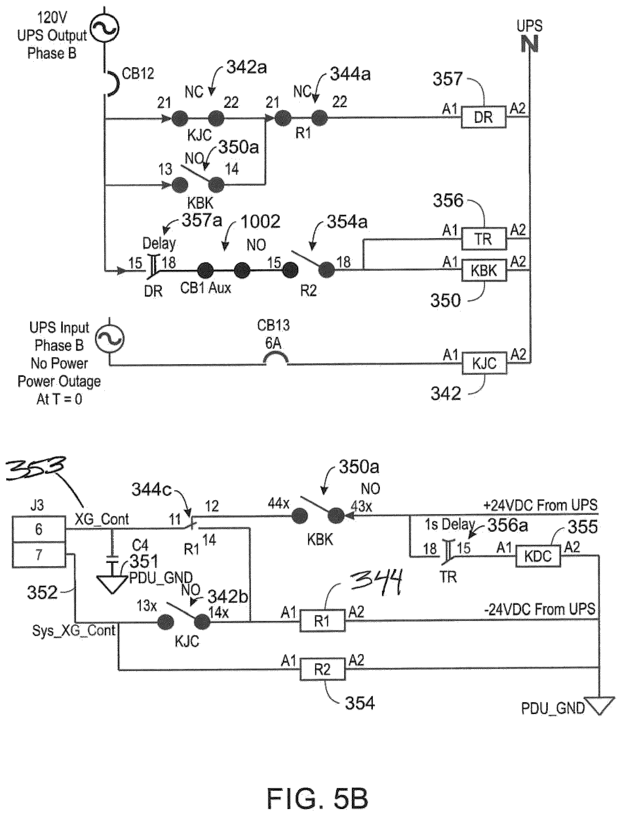

Upon detection of a power loss, the system begins power loss mode or operations. FIGS. 5A-8B are schematic diagrams depicting a series of operations of the power circuitry and control circuitry during the power loss mode. Power loss mode begins in FIG. 5A with the detection of the loss of power from the primary power source 122. FIG. 5B depicts the control circuitry during this initial phase of power loss mode. In this initial phase, the Aux contact of CB1 1002 is closed. Shortly after power loss, when no power is being supplied to the UPS 124, KJC coil 342 is de-energized. Auxiliary contacts 342a and 342b are closed and opened respectivily, interrupting the XG_ CONT signal 353 to the KSS and KXG contacts 314, 315, as depicted in FIG. 5A. Additionally, the R1 contact 344 is switched (i.e., 344b is switched to 344c). As shown in FIG. 5B, in the control circuitry, the KJC and R1 contacts 342a, 344a are closed. FIG. 5A depicts the KSS and KXG contacts 315, 314 being opened as a result of the interruption of the SYS_XG_CONT signal 352, which also prevents power transmission to the HVDC load 320. The SYS_XG_CONT signal 352 is no longer being transmitted from the GCB, as shown in FIG. 5B. Additionally, the R2 354a contact is opened in the control circuitry, as depicted in FIG. 5B.

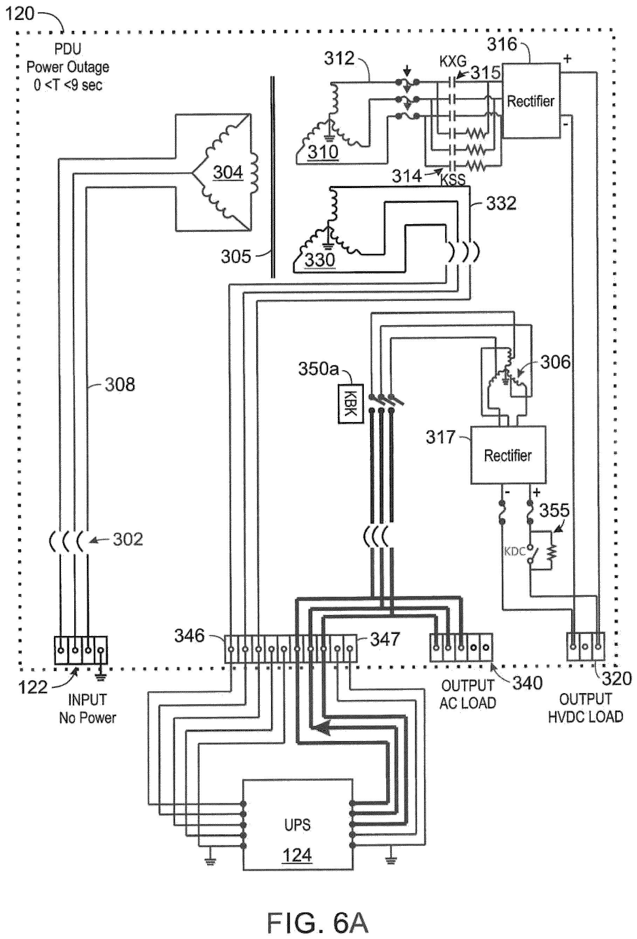

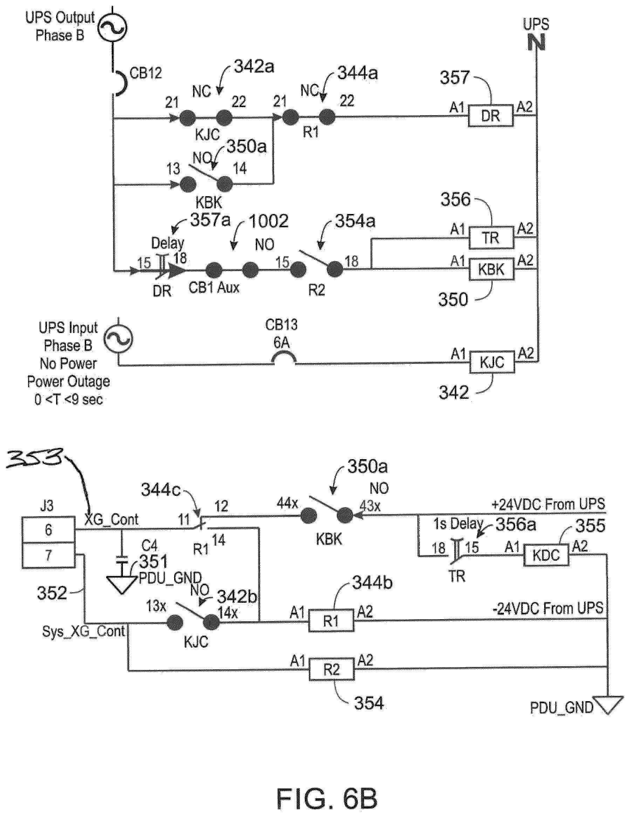

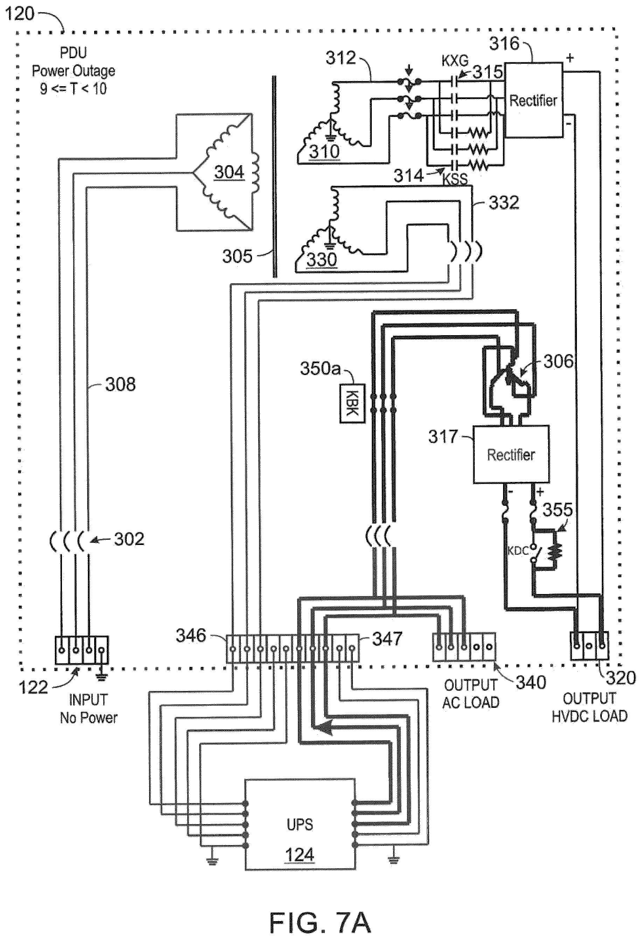

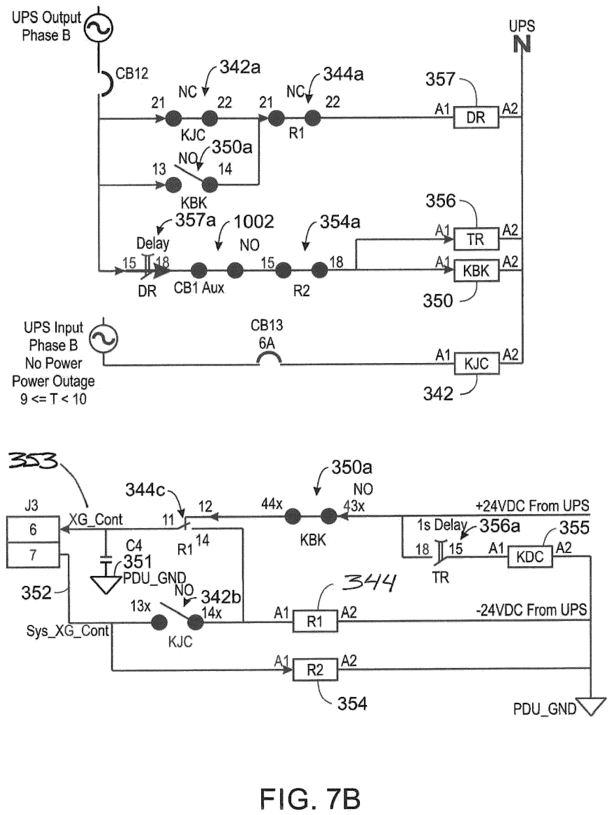

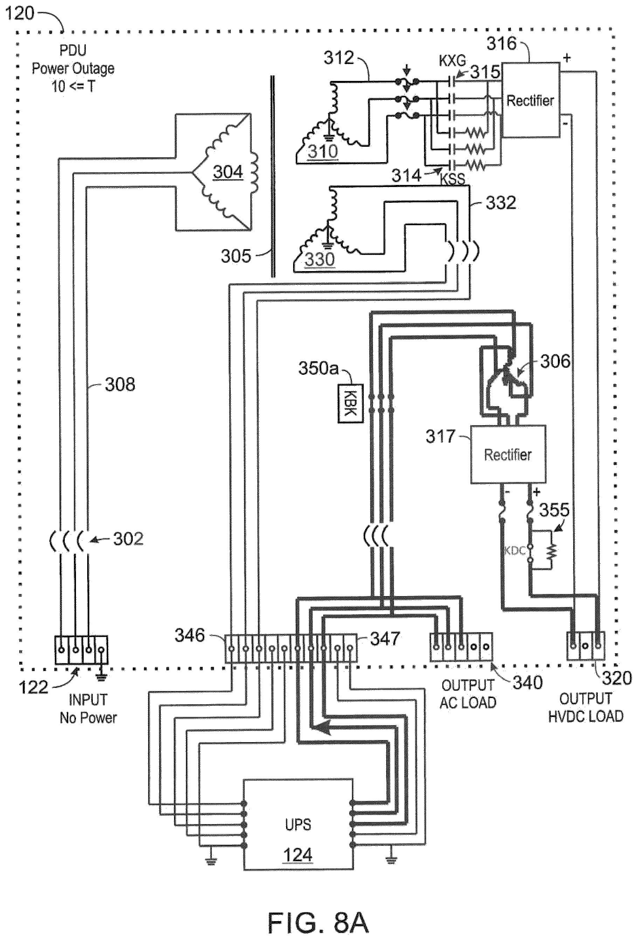

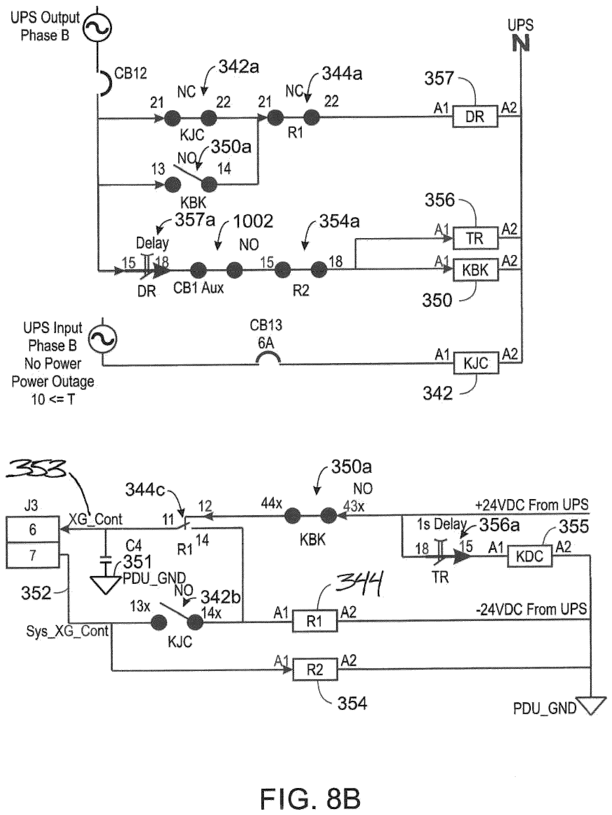

In FIG. 6A, the UPS battery power is presented to the PDU TS5 347 or AC load 340, thus supplying power to the gantry and the related control circuitry, as shown in FIG. 6A and 6B. FIGS. 7A and 7B depicts the power and control circuitry when a DR timer 357 has been energized. The DR timer 357 is a delay circuit enabled to determine whether the power loss detected by the KJC contact switch 344a is a momentary power loss (e.g., a power flicker or surge) or a significant power loss (e.g., a power loss lasting more than a threshold amount of time). The DR timer 357 enables a delay of the threshold amount of time or threshold period of time, which in some examples is 9 seconds. After the delay, the DR contact 357a status changes. After a time delay (e.g., 12.5 seconds after the primary power source is lost), the SYS_XG_CONT signal 352 from the GCB resumes, and as a result, the R2 relay 354 is energized and R2 NO contact 354a is closed, and the KBK contact 350 and timer TR 356 are energized, and immidiatelly KBK contact 350a is closed, enabling an autotransformer 306 to boost the voltage, then rectified, enabling power to be provided from the UPS 124 to the DC load 320, as shown in FIGS. 8A and 8B. In the illustrated example, the UPS 124 is sufficient to provide HVDC power to the DC load 320 for long enough period of time to cool the tube. In other applicaitons, the UPS 124 may be adjusted to provide a different amount of power. Additionally, the KSS and KXG contacts 315, 314 are closed by sending 24V signal through KBK Contact 350a and Relay R1 344c as XG_CONT 353 signal. After a 1 second delay, the TR contact 356a status changes, closing the KDC contact 355a and enabling power transmission from the UPS 124 to the recitfier 317 and the DC load 320 to provide backup HVDC power to the CT imaging system. In some examples, the UPS 124 is able to provide backup HVDC power until the power from the power source 122 resumes. Alternatively, the UPS 124 provides power for a designated time period (e.g., 45 minutes) to enable the CT imaging system to properly cool down and safely shut down.

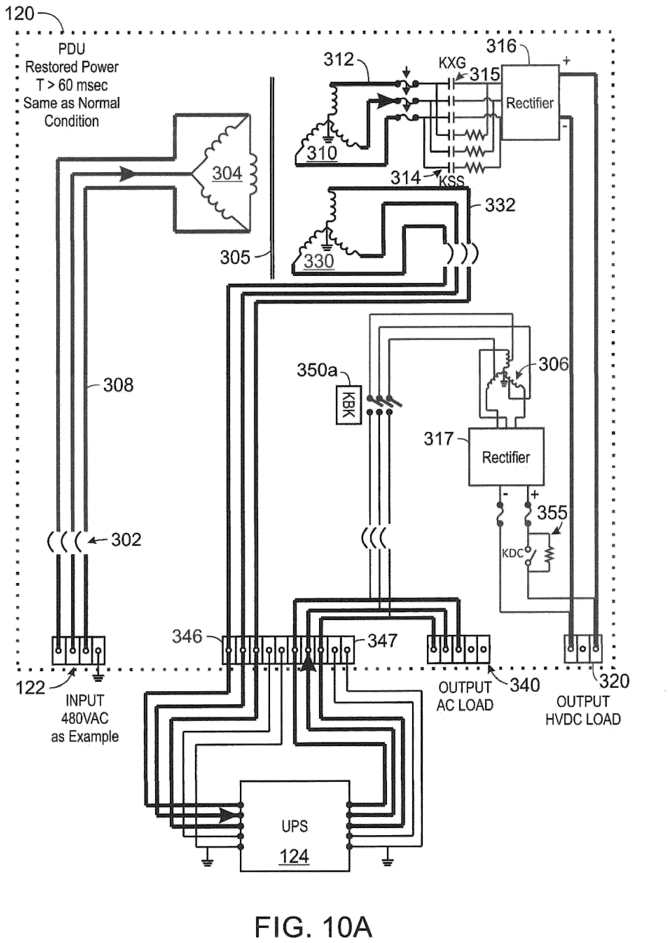

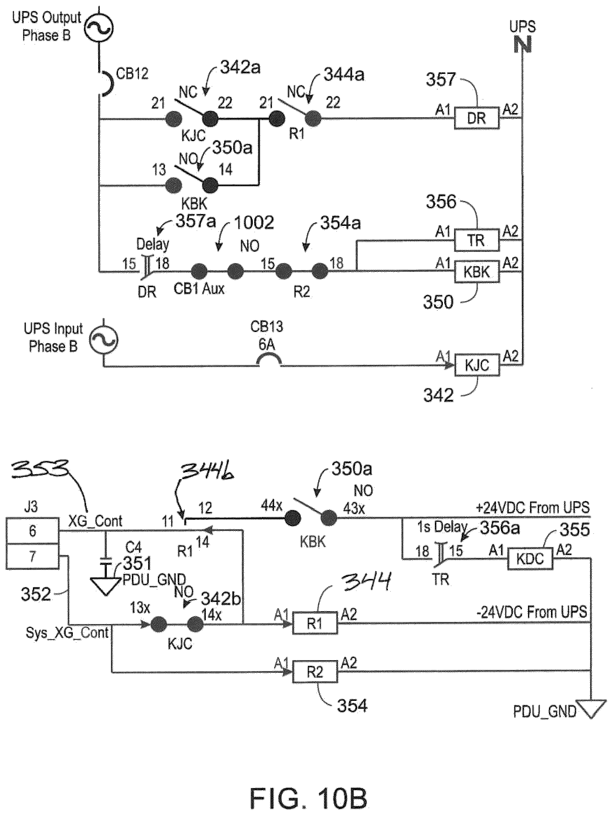

FIGS. 9-10B show schematic diagrams of a series of operations of the power circuitry and control circuity power return conditions. FIG. 9 depicts the initial return of power from the power source. Upon return on the power source, the LVAC power is supplied via the primary power source 122 rather than via the UPS 124, as shown in FIG. 9. For few milliseconds the DC load 320 has two power sources. One from the power source 122 through the secondary winding 305, KSS 314 and KXG 315 contactors and rectifier 316. The second source is from the UPS 124 through KBK 350a contactor, autotransformer 306 and rectifier 317. Haviang two available power sources prevents the CT system 102 and computing device 216 from experiencing any sag or drop voltage. The backup power will switch to main power source without resetting or rebooting the CT system. Additionally, or alterantavietly, in some examples, a capatitor 351 is positioned in the control circuit between the R1 contact 344a and the XG_CONT signal 353. The capacitor holds sufficient charge to overcome minor power disruptions, such as switching form the UPS 124 back to the primary power source 122. By overcoming these minor power disruptions, the capacitor 351 can prevent the need to reboot the CT system 102, enabling the CT system 102 to return to scan mode more quickly. Additionally, the normal SYS_XG_CONT signal 352 is supplied from the GCB. The KBK 350a and KDC 355a contacts are opened, preventing power supply from the UPS 124 to the HVDC ouput 320, changing the backup HVDC status. Contacts KJC Aux 342b and R1 344a are closed, enabling the SYS_XG_CONT signal 352 to reach the KSS and KXG contacts 315, 314, as shown in FIG. 10A and 10B. The KSS contact 314 is closed, followed after a 1.2 second delay by the KXG contact 315 being closed. Normal condition operations are resumed, and the CT imaging system is ready for operation.

FIG. 11 depicts a method 1100 for adjusting an energy source based on an availability of electrical energy from a main power source 122. Instructions for the method may be executed by and stored on memory of a controller of the PDU (e.g., PDU controller 130 of FIG. 1). The controller may be configured to receive inputs from one or more sensors of the PDU 120 and adjust operation of one or more switches to change the direction of current flow.

The method 1100 begins at 1102, which inlcudes turning the CT imaging system power on. Turning the system power on may include, for example, operating a switch and/or a plug. After the system power is turned on, the CT imaging system proceeds to initiate normal operating conditions to prepare to be ready to perform scans. In some examples, immediately upon turning on the system power, AC power is available to the AC load via an AC power output of the PDU, as shown in FIGS. 3A and 3B. The method continues to step 1104, and the GCB sends the SYS_XG_CONT signal to the PDU after closing KJC contact, initiating the startup process for normal operating conditions (block 1106). Initiating normal operating conditions may include closing the R2, R1 and KSS contacts, as shown in FIGS. 4A and 4B. Additionally, the KXG connector is closed after a delay and HVDC power is provided to the gantry. The system is now at normal opening conditions and ready to perform scans (block 1108). During normal operating conditions, the UPS may be charged.

In block 1110, the system (e.g., the gantry control board, the PDU controller, etc.) is operative to detect a power outage. If no power outage is detected, the system continues operating under normal conditions. If a power outage is detected, the system initiates power loss operating conditions (block 1112). Initially, the KJC auxiliary contact is opened the SYS_XG_CONT signal is interrupted (FIG. 5A). This causes the KSS and KXG contacts to open, disabling the HVDC output from the PDU (FIG. 5A). Simultaneously, UPS power is provided as the AC output from the PDU. A delay relay is enabled. In block 1114, the system determines (e.g., using a timed delay relay) if the power outage has lasted for a time exceeding a threhold. If the power outage has not exceeded a threshold amout of time, the system identifies the power outages as a temporary surge or flicker and returns to normal operating conditions. If the time of the power outage has exceeded the threshold, the delay relay contact DR closes, and the system continues in power loss mode operating conditions (block 1116). To continue on power loss mode, the system resends the SYS_XG_CONT signal via the GCB, which is now powered using the UPS. The SYS_XG_CONT signal closes KSS contact, then the KXG contact, and simultaneously closes the KBK contact and enables the timer relay. As a result of closing the KBK relay, a transformer is energized (FIGS. 8A and 8B). The timer relay delays the close of the KDC contact by 1 second, then closes the KDC contact, enabling HVDC power to be supplied from the UPS to the DC ouput of the PDU.

The method continues at block 1118 by waiting for the tube of the imaging system to cool. In some examples, the system waits a predetermined amount of time (e.g., 45 minutes) for the tube to cool completely. In other examples, sensors may be used to determine when the tube has cooled completely. When the tube is cool, the method continues at block 1120 by determining if the primary power source has been restored. If the primary power source has been restored, the system initiates a return to normal operating conditions (block 1122). The return to normal operating conditions begins with the LVAC power is supplied via the primary power source rather than via the UPS, as shown in FIG. 9. Additionally, the normal SYS_XG_CONT signal is supplied from the GCB. The KBK and KDC contacts are opened, preventing power supply from the UPS to the HVDC ouput, changing the backup HVDC status. Contacts KJC Aux and R1 are closed, enabling the SYS_XG_CONT signal to reach the KSS and KXG contacts, as shown in FIG. 10A and 10B. The KSS contact is closed, followed after a 1.2 second delay by the KXG contact being closed. This enables the normal supply of the HVDC power via the primary power source, as shown in FIGS. 10A and 10B. The system is again ready to scan and the method is complete.

If at block 1120, the primary power source has not been restored, the tube cooling is complete (block 1124) and the system shuts down and the method is complete.

As used herein, an element or step recited in the singular and proceeded with the word "a" or "an" should be understood as not excluding plural of said elements or steps, unless such exclusion is explicitly stated. Furthermore, references to "one embodiment" of the invention do not exclude the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

The control methods and routines disclosed herein may be stored as executable instructions in non-transitory memory and may be carried out by the control system including the controller in combination with the various sensors, actuators, and other engine hardware. The specific routines described herein may represent one or more of any number of processing strategies such as event-driven, interrupt-driven, multitasking, multi-threading, and the like. As such, various actions, operations, and/or functions illustrated may be performed in the sequence illustrated, in parallel, or in some cases omitted. Likewise, the order of processing is not necessarily required to achieve the features and advantages of the example embodiments described herein, but is provided for ease of illustration and description. One or more of the illustrated actions, operations and/or functions may be repeatedly performed depending on the particular strategy being used. Further, the described actions, operations and/or functions may graphically represent code to be programmed into non-transitory memory of the computer readable storage medium in the engine control system, where the described actions are carried out by executing the instructions in a system including the various engine hardware components in combination with the electronic controller.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A system, comprising:
a power distribution unit (PDU) configured to receive power from a main power source and an uninterruptible power supply (UPS), wherein the UPS is configured to directly power an output alternating current (AC) load after the main power source in unavailable, and the UPS is further configured to power an output high voltage direct current (HVDC) load via passing current through an autotransformer to boost the voltage and an AC/DC converter.

2. The system of claim 1, further comprising a first contact switch to detect main power availability, wherein when main power is available, the first contact switch is in a first position to enable a signal to enable normal operating conditions, and wherein when main power is not available, the first contact switch is in a second position to enable power loss mode operating conditions.

3. The system of claim 2, further comprising a delay circuit, wherein the delay circuit is enabled to determine whether the power loss detected by the first contact switch is a momentary power loss or a significant power loss.

4. The system of claim 3, wherein the delay circuit includes a timer which closes a delay contact after a threshold period of time.

5. The system of claim 4, further comprising a second contact, wherein the second contact closes after the delay contact closes if the power loss exceeds a threshold period of time, and wherein closing the second contact enables the UPS to provide power to the autotransformer.

6. The system of claim 1, wherein the output HVDC load supplies HVDC power to a plurality of systems within a gantry of a computed tomography (CT) imaging system.

7. The system of claim 1, wherein the UPS is configured to power the output AC load and the output HVDC load in response to power from the main power source being unavailable.

8. The system of claim 1, further comprising a capacitor to hold sufficient charge to overcome power disruptions and prevent the need to reboot the system.

9. A computed tomography (CT) imaging system, comprising:
a gantry coupled to an output HVDC load;
a power cabinet coupled to an output AC load;
a power distribution unit (PDU);
wherein the PDU is configured to receive power from one of a main power source and an uninterruptible power supply (UPS), wherein the UPS provides power to the HVDC load via an autotransformer when the main power source is unavailable;
a plurality of contactors and a timer coupled to the PDU and UPS; and
a controller with computer readable instructions stored on memory thereof for controlling the plurality of contactors based on availability of the main power source and an input from the timer.

10. The CT imaging system of claim 9, wherein the plurality of contactors includes a first contact switch to detect main power availability, wherein when main power is available, the first contact switch is in a first position to enable a signal to enable normal operating conditions, and wherein when main power is not available, the first contact switch is in a second position to enable power loss mode operating conditions.

11. The CT imaging system of claim 10, further comprising a delay circuit, wherein the delay circuit is enabled to determine whether the power loss detected by the first contact switch is a momentary power loss or a significant power loss.

12. The CT imaging system of claim 11, wherein the delay circuit includes a timer which closes a delay contact after a threshold period of time.

13. The CT imaging system of claim 12, wherein the plurality of contactors further includes a second contact, wherein the second contact closes after the delay contact closes if the power loss exceeds a threshold period of time, and wherein closing the second contact enables the UPS to provide power to the autotransformer.

14. The CT imaging system of claim 9, wherein the output HVDC load supplies HVDC power to a plurality of systems within a gantry of a computed tomography (CT) imaging system.

15. The CT imaging system of claim 9, further comprising a capacitor to hold sufficient charge to overcome power disruptions and prevent the need to reboot the CT imaging system.
